# EUROPÄISCHE PATENTSCHRIFT

(11) **EP 0 995 441 B1**
(45) Veröffentlichungstag und Bekanntmachung des Hinweises auf die Patenterteilung: **24.07.2002**
(21) Anmeldenummer: 99118622.2
(22) Anmeldetag: 21.09.1999
(51) Int. Cl.: A61K 31/505, A61K 31/165

(54) **Medikamentenkombination zur Therapie der erektilen Dysfunktion**
Combination of compounds for treating erectile dysfunctions
Combination des médicaments pour la therapie des dysfonctionnements erectiles

(30) Priorität: 25.09.1998 DE 19844162
(43) Veröffentlichungstag der Anmeldung: 26.04.2000
(73) Patentinhaber: PD Dr. Dunzendorfer, 60528 Frankfurt/M (DE)
(72) Erfinder: PD Dr. Dunzendorfer, 60528 Frankfurt/M (DE)

(56) Entgegenhaltungen:
- WO-A-92/03141
- WO-A-99/30697
- US-A- 5 059 603
- US-A- 5 145 852
- SOLI ET AL: "Vasoactive cocktails for erectile dysfunction: chemical stability of PGE1, papaverine and phentolamine" JOURNAL OF UROLOGY,US,BALTIMORE, MD, Bd. 160, Nr. 160, August 1998 (1998-08), Seiten 551-555-555, XP002125439 ISSN: 0022-5347
- BECKER A J ET AL: "[Pharmacological therapy of erectile dysfunction]. Pharmakologische Therapie der erektilen Dysfunktion." UROLOGE. AUSGABE A, (1998 SEP) 37 (5) 503-8. REF: 43, XP002135980

## Beschreibung

Die Erfindung betrifft die Bereitstellung eines Medikaments zur oralen Therapie der erektilen Impotenz.

Im Stand der Technik ist es bekannt, eine erektile Impotenz durch hohe Einzelgaben von Sildenafil zu therapieren, wobei jedoch cardiotoxische und/oder weitere Nebenwirkungen zu beobachten sind. Zusätzlich gibt es bestimmte Patientengruppen, bei denen eine Therapie mit Sildenafil nicht anspricht.

"Der Urologe" (5-1998; 37:503-508) beschreibt Kombinationen aus alpha-Adrenozeptor-Antagonisten (z.B.Yohimbin; Phentolamin) und Sildenafil zur Therapie der Impotenz.

US 5 145 852 offenbart eine Injektionslösung bestehend u.a. aus alpha-Adrenozeptor-Antagonisten und dem Phosphosiesterasehemmer Dipyridamol.

"Journal of Urology" (1998; vol. 160; 551-555) referiert über sogenannte "vasoaktive Cocktails" zur Therapie der erektilen Dysfunktion, bestehend aus den vasodilatierend wirksamen Substanzen Papaverin, Phentolamin und Prostaglandin PGE1.

US 5 059 603 offenbart eine Kombiation zur Therapie von Impotenz bestehend aus Vasodilatatoren (z.b. alpha-Adrenozeptor-Antagonisten) und Vasokonstriktoren (z.B. Epinephrin oder Koffein).

WO 92 03141 A1 verweist auf vasoaktive Kombinationen zur Therapie von Impotenz, bestehend aus z.B. den Vasokonstiktoren Epinephrin, Norepinephrin und den alpha-Adrenozeptor-Antagonisten Yohimbin und Prazosin.

Der Erfindung liegt daher die Aufgabe zugrunde, ein Medikament zur oralen Therapie der erektilen Impotenz zu schaffen, das die cardiotoxischen und/oder weitere Nebenwirkungen reduziert und mit dem auch Patienten therapierbar sind, die auf den Wirkstoff Sildenafil allein nicht ansprechen.

Erfindungsgemäß wird diese Aufgabe durch eine Medikamentenkombination gelöst, umfassend 10 bis 100 mg Sildenafil und in therapeutisch wirksamer Menge einen weiteren Wirkstoff zur Verbesserung der Durchblutungsverhältnisse und zur Erhöhung der cyclischen Adenosinmonophosphat- und cyclischen Guanosinmonophosphat-Spiegel und des Gewebespiegels NO in den Corpora cavernosa.

Die Unteransprüche 2 bis 14 geben vorteilhafte Ausgestaltungen solch einer Medikamentenkombination nach Anspruch 1 wieder.

Gemäß Anspruch 15 kann die Medikamentenkombination in Form von Tabletten, Dragees, Hautcremes, Lotionen und Injektionslösungen verwendet werden.

Überraschenderweise erwies sich in klinischen Studien, daß Patienten mit erektiler Impotenz (ED) nach Therapieversagen unter der Behandlung mit Sildenafil allein (ED-SN negativ nach Sildenafil-Therapie in einer Menge von 100 mg Sildenafil) mit der erfindungsgemäßen Medikamentenkombination erfolgreich therapierbar waren.

Die erektile Dysfunktion wird durch eine Reihe von sogenannten Grunderkrankungen, wie Diabetes, Krebsbehandlung mit Zytostatika, Nierenschäden, Gefäßerkrankungen, Depressionen, Unfällen im Wirbelsäulenbereich, Zuständen nach Prostata-Operationen und ähnlichem aufgelöst, und führt zu komplexen Störungen im Bereich der Durchblutung der Corpora cavernosa. Dabei sind in der Regel nicht nur Störungen des Blutzuflusses in die Corpora cavernosa, Störungen in der Freisetzung oder Umsatzrate von Stickoxiden (NO) mit mangelnder Steigerung des cyclischen Guanosinmonophosphat-Spiegels und Ausbleiben der Erschlaffung der glatten Muskulatur der Corpora cavernosa festzustellen, sondern ebenso ein extrem hoher Abstrom über die Venengebiete im Bereich der prostatischen Harnröhre, der Blase und im sogenannten kleinen Becken.

Überraschenderweise wurde festgestellt, daß mit der erfindungsgemäßen Medikamentenkombination gleichzeitig der venöse Abfluß aus der Corpora cavernosa und/oder aus dem pelvinen Bereich verringert werden kann und dabei direkt oder indirekt der Gewebespiegel für cyclisches Guanosinmonophosphat und der Gewebespiegel Adenosinmonophosphat und der Gewebespiegel NO in den Corpora cavernosa erhöht wird. Der Wirkungsmechanismus der Medikamentenkombination besteht daher in einer verringerten venösen Abflußphase aus dem Schwellkörpergebiet bei gleichzeitiger Erhöhung der Gewebekonzentration von Stickoxiden, die unter anderem durch die Hemmung der Phosphodiesterase Typ 5 der Corpora cavernosa bedingt ist.

Es wurde eine Vielzahl von Medikamentenkombinationen untersucht und aus der radiologischen Diagnostik von Cavernosogrammen ergaben sich deutlich reduzierte Venenabflüsse aus dem Erektionsgebiet der Corpora cavernosa bei Verwendung der erfindungsgemäßen Medikamentenkombination.

So wurde beispielsweise bei Verwendung einer Medikamentenkombination, bestehend aus Sildenafil der chemischen Strukturformel: 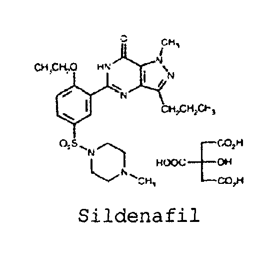 im erfindungsgemäßen Mengenbereich und 10 µg Suprarenin der Strukturformel: 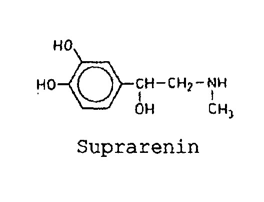 als α-Sympathicomimetikum unter endoskopischer Beobachtung nach Injektion in die Corpora cavernosa bei Patienten mit Priapismus im Bereich der prostatischen Harnröhre eine signifikante Zunahme des Muskeltonus in Sphincter internus und im Sphincter externus Bereich registriert. Durch fiberoptische Druckmessungen traten hier um 195-215% höhere Druckwerte auf. Diese pharmakologischen Veränderungen sind ein indirekter Hinweis auf die alterierte Gefäßabflußsituation im Funktionsbereich der angrenzenden Organe an die Corpora cavernosa. Bei einer Vergleichsgruppe, die nach 100 mg Sildenafil eine klinisch suffiziente Erektion zeigte, ließen sich im Bereich des Sphincter internus sowie des Sphincter externus keine vergleichbaren hohen Druckwerte feststellen.

Tabelle 1 zeigt die Wirkung einer Medikamentenkombination, bestehend aus Sildenafil und Midodrin der chemischen Strukturformel: 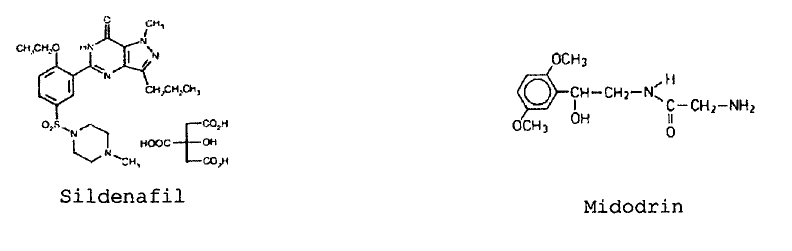 wobei die Sildenafil-Mengen von 10 bis 100 mg und die Midodrin-Mengen von 5 bis 25 mg variiert wurden.

Bei 10 Patienten mit ED-SN im Alter zwischen 45 bis 65 Jahren ohne Nitropräparate und koronare Herzkrankheiten lag beispielsweise die Ansprechrate der Medikamentenkombination, bestehend aus 50 mg Sildenafil und 25 mg Midodrin bei 7 von 10 Patienten, wenn die Medikamentenkombination in Form von Zwei-Kompartment-Dragees gegeben wurde.

Bei Gabe der Medikamentenkombination in einer üblichen Retard-Form gelang es, die Ansprechrate auf 8 von 10 Patienten zu erhöhen.

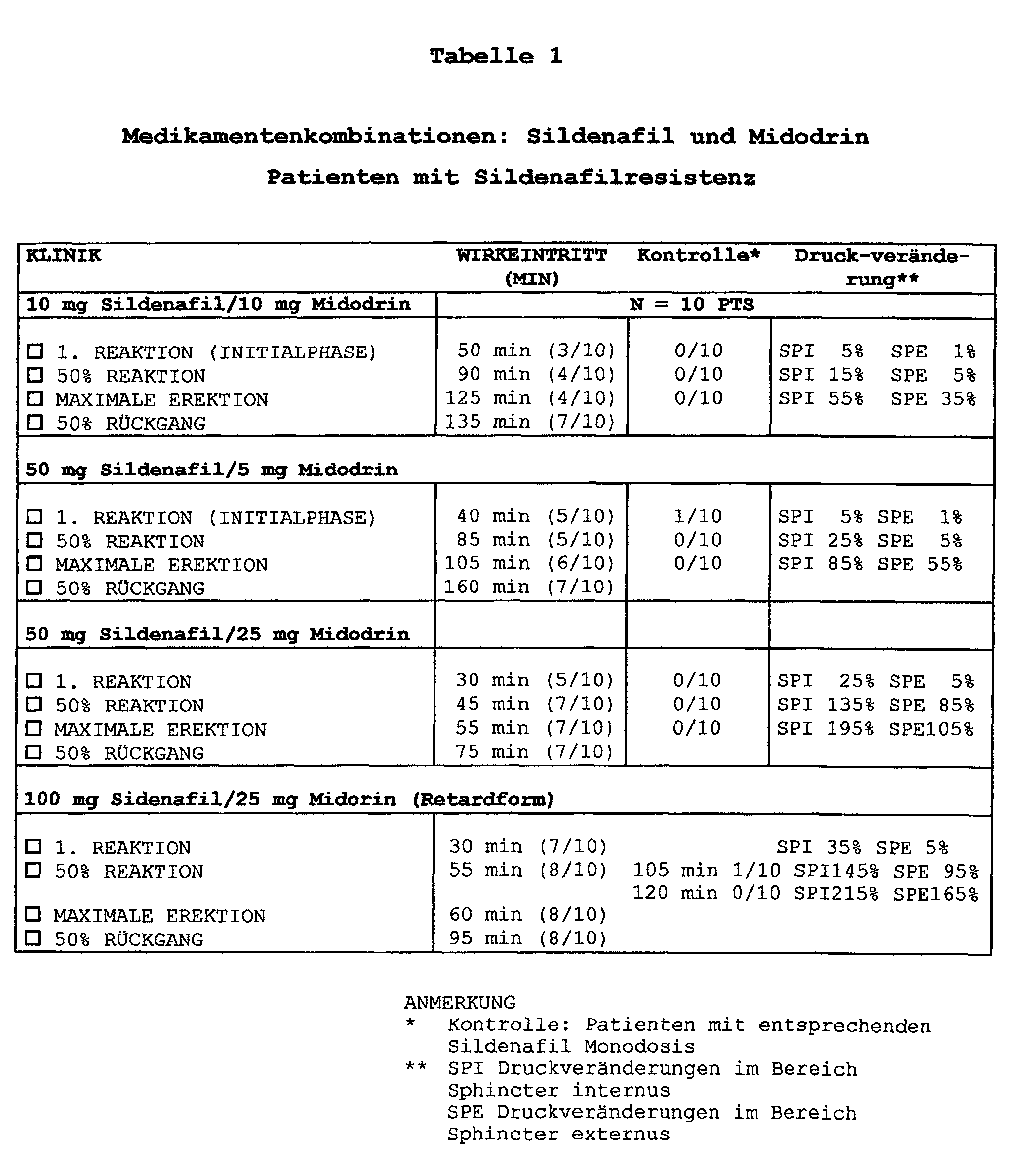

Die erfindungsgemäßen Medikamentenkombinationen können neben üblicherweise verwendeten Bestandteilen zusätzlich zu den Wirkstoffen noch übliche Hüll- und/oder Mantelkomponenten enthalten.

Diese Hüll- und/oder Mantelkomponenten sind in bestimmten prozentualen Anteilen entsprechend den Mischungsverhältnissen Speichel-, Magen-, Duodenal- und Dünndarmsaft-resistent und ermöglichen somit eine steuerbare Freisetzung der Einzelkomponenten.

Bei einer weiteren vorteilhaften Ausgestaltung der erfindungsgemäßen Medikamentenkombinationen können die Wirkstoffe in Hilfsmittel integriert sein, so daß die Freisetzung der Wirkstoffe zeitlich verzögert erfolgt.

Überraschenderweise wurde festgestellt, daß nicht nur eine Mischung der Wirkstoffe in den erfindungsgemäßen Medikamentenkombinationen wirksam ist, sondern daß auch Syntheseprodukte, die aus Sildenafil und den weiterhin verwendeten Wirkstoffen hergestellt sind, erfolgreich eingesetzt werden können. Bei diesen Syntheseprodukten wird Sildenafil mit dem weiteren Wirkstoff vorzugsweise über eine Ester- oder Amidverbindung synthetisch verknüpft. Medikamentenkombinationen, welche solche Syntheseprodukte enthielten, zeigten vergleichbare Wirkungen für die entsprechenden Untersuchungsgruppen.

Bei einer weiteren vorteilhaften Ausgestaltung der erfindungsgemäßen Medikamentenkombination werden die Wirkstoffe aus der Kapsel zeitlich verzögert herausgelöst und die Kapsel wird dabei wirkstofffrei ausgeschieden. Vorteilhafterweise wird der Wirkstoff im Austausch mit wäßriger Lösung aus den Magen- und Darmsäften ausgetauscht.

Folgende Medikamentenkombinationen erwiesen sich als wirksam, wobei die Wirkstoffe in unten angegebenen Mengen enthalten waren.

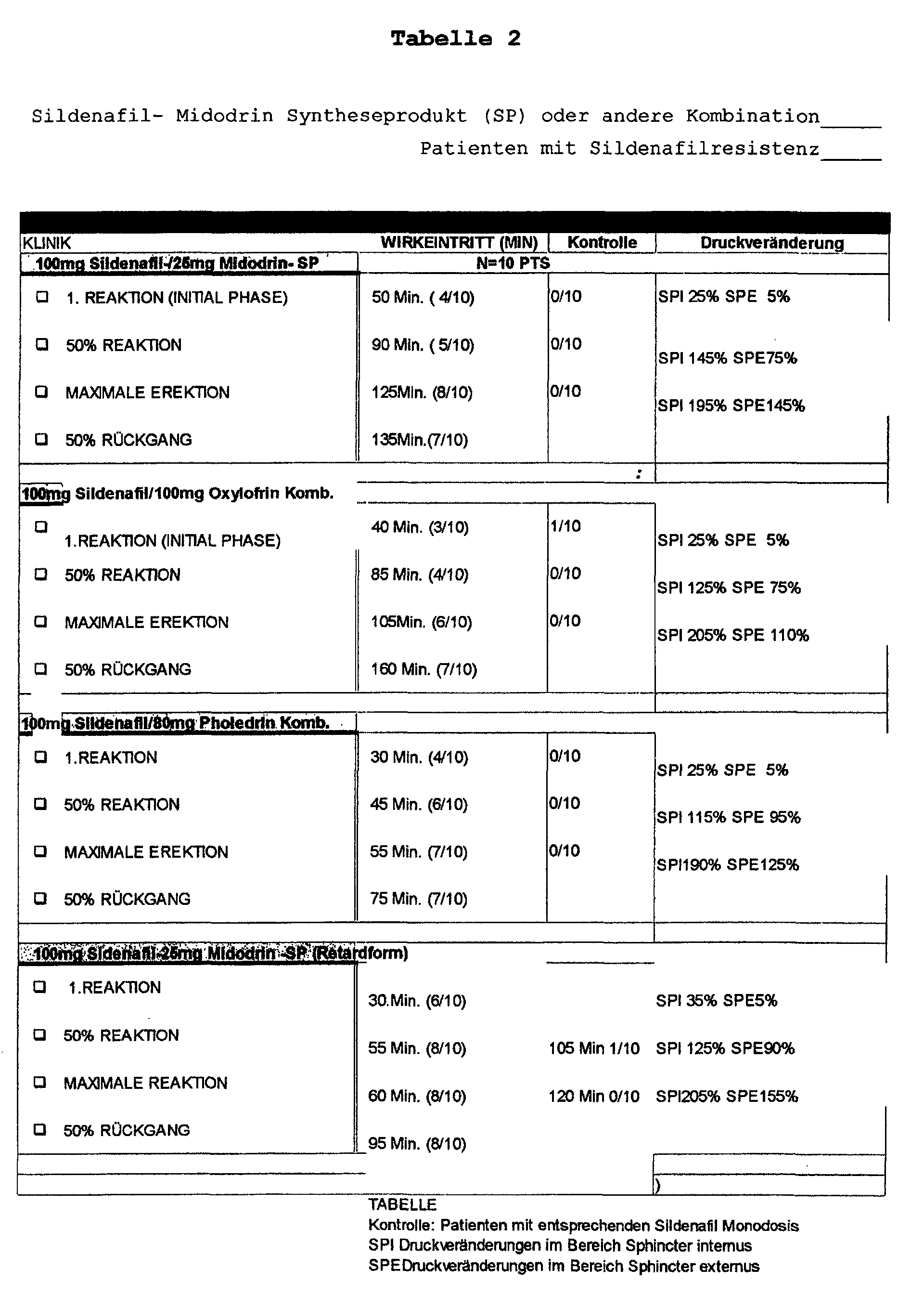

1. 10 bis 100 mg Sildenafil und 5 bis 100 mg Midodrin mit folgender chemischer Strukturformel: 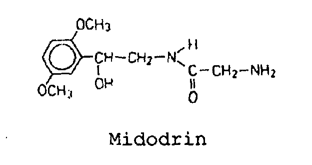
2. Syntheseprodukt aus Sildenafil und Midodrin mit folgender Strukturformel:
3. 10 bis 100 mg Sildenafil und systemisch anwendbare α-Sympathicomimetika 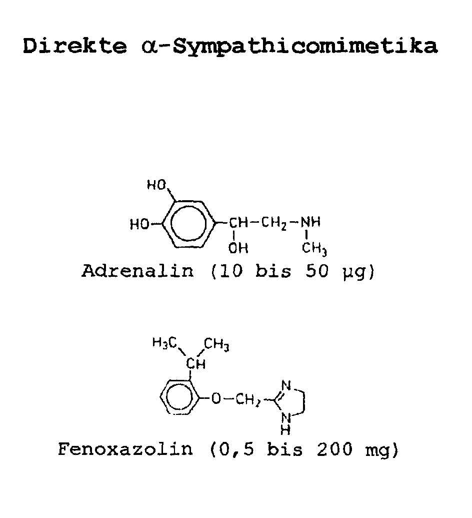 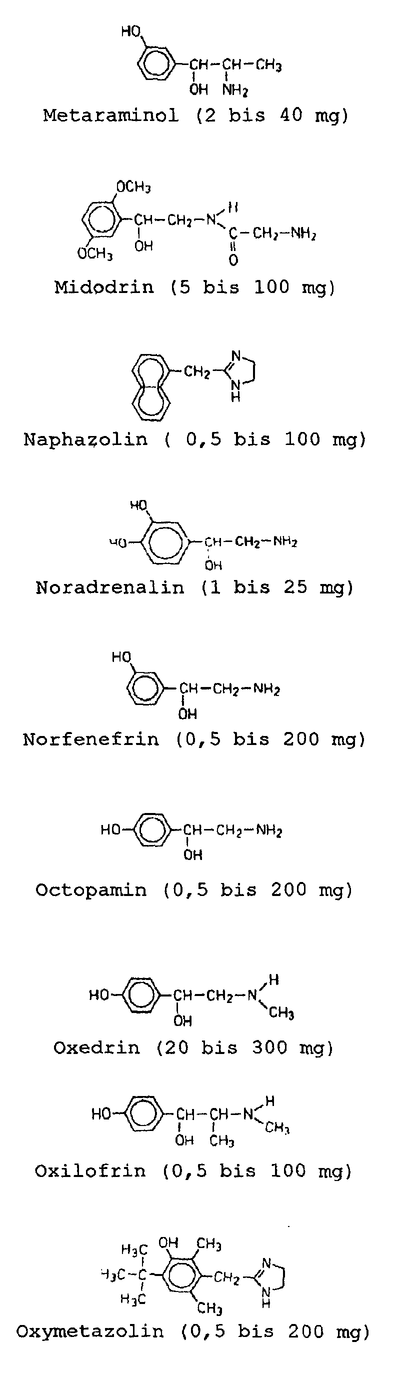 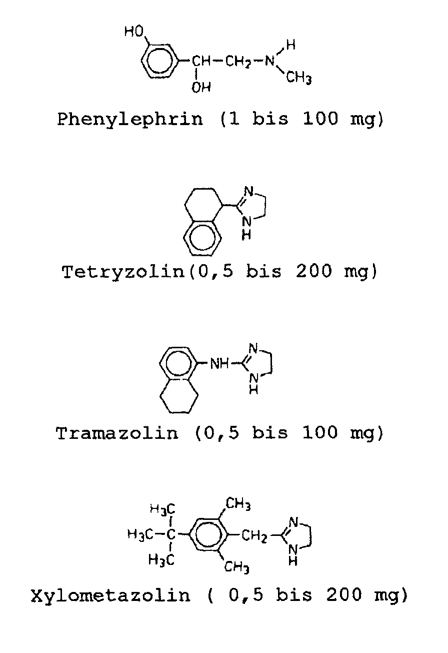 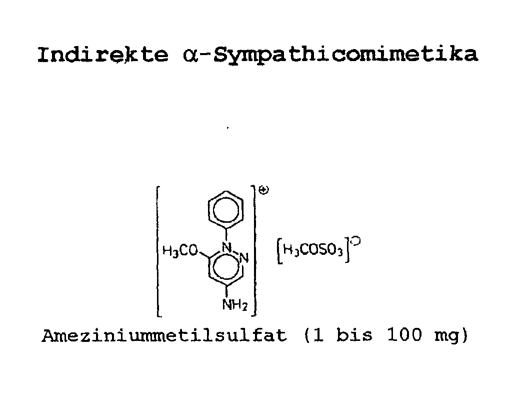 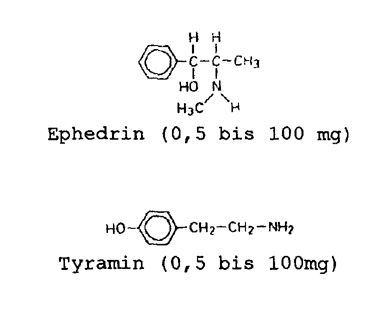
4. 10 bis 100 mg Sildenafil und 0,5 bis 50 mg Etilefrin mit der chemischen Strukturformel: 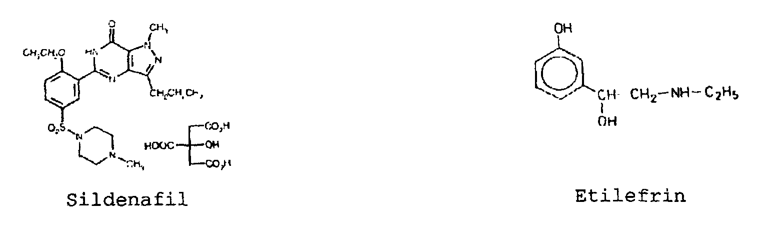
5. 10 bis 100 mg Sildenafil und 0,5 bis 100 mg Oxylofrin mit der chemischen Strukturformel: 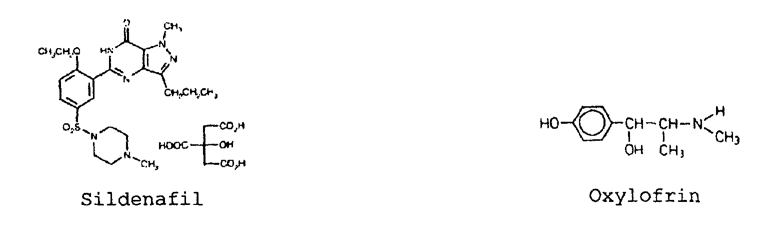
6. 10 bis 100 mg Sildenafil und 1 bis 100 mg Pholedrin mit der chemischen Strukturformel: 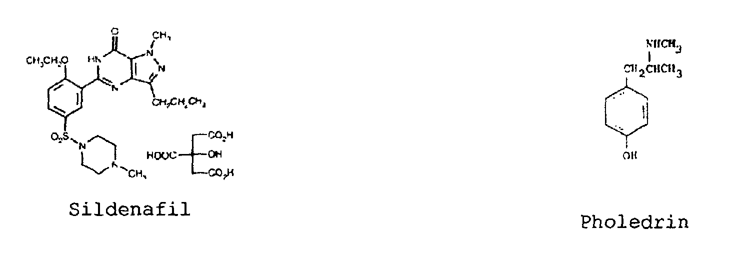
7. 10 bis 100 mg Sildenafil und 0,2 bis 10 mg Ergotoxine sowie deren Dihydro-Derivate mit der chemischen Strukturformel: 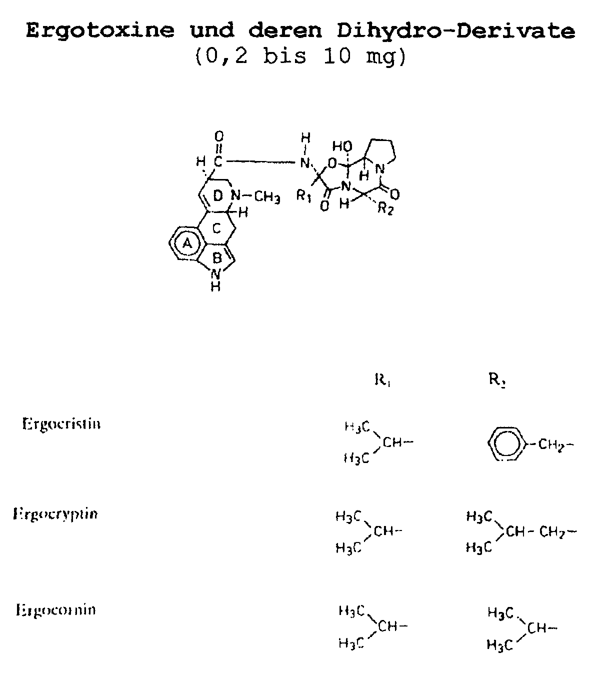
8. 10 bis 100 mg Sildenafil und 0,5 bis 200 mg Norfenefrin mit der chemischen Strukturformel:

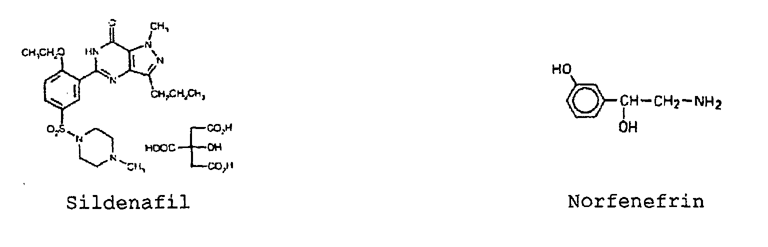

Im typischen Behandlungsfall von ED und ED-SN werden die erfindungsgemäßen Medikamentenkombinationen 30 bis 90 Minuten vor Wirkeintritt eingenommen, wobei die Wirkung nach weiteren 160 Minuten nicht mehr nachweisbar ist.

Aus den Wirkkinetiken ist zu folgern, daß die Kombinationspräparate nach 45 bis 90 Minuten die maximale Plasma- und Gewebekonzentration erreicht haben, wobei die Resorptionszeit im Mittel bei 65 +/- 25 Minuten liegt. Bei der Retardform sind die Werte für die maximale Plasma- und Gewebekonzentration in Abhängigkeit von der Retardierungskomponente meßbar und somit steuerbar. Daraus ergab sich auch ein verlängerter Wirknachweis von 25 +/- 10 Minuten.

Pharmakologisch wird durch die venöse Vasokonstriktion der Venenabfluß reduziert, und die Tonuserhöhung der Muskulatur im Bereich der prostatischen Harnröhre wirkt sich direkt und indirekt auf die blutgefüllten Corpora cavernosa aus. Biochemisch kommt es zu einer Aktivierung der Adenylatcyclase, wobei der konsekutive Umbau zu Adenosinmonophosphat durch die Phosphodiestease inhibiert wird, so daß gleichzeitig hohe cyclische Adenosinmonophosphat- und cyclische Guanosinmonophosphat-Spiegel in den Corpora cavernosa entstehen, die zu einer potenzierten und prologierten Erschlaffung der glatten Muskulatur führen und den Bluteinstrom in die Corpora cavernosa steigern.

## Patentansprüche

1. Medikamentenkombinationen zur Herstellung eines Arzneimittels für die Behandlung der erektilen Dysfunktion bestehend aus einer Substanz, die die Phosphodiesterase 5 hemmt und einer zweiten Substanz, die ein alpha Sympathicomimetikum darstellt.

2. Medikamentenkombinationen zur Herstellung eines Arzneimittels für die Behandlung der erektilen Dysfunktion gemäss Anspruch 1, wobei der Phosphodiesterase 5 Inhibitor aus Sildenafil besteht und die zweite Substanz aus einem alpha Sympathicomimetikum besteht.

3. Medikamentenkombinationen zur Herstellung eines Arzneimittels für die Behandlung der erektilen Dysfunktion gemäss Anspruch 1, wobei der Phosphodiesterase 5 Inhibitor aus Sildenafil besteht und die zweite Substanz aus dem alpha Sympathicommietikum Midodrin besteht.

4. Medikamentenkombinationen zur Herstellung eines Arzneimittels für die Behandlung der erektilen Dysfunktion gemäss Anspruch 1, wobei der Phosphodiesterase 5 Inhibitor aus Sildenafil besteht und die zweite Substanz aus dem alpha Sympathicominetikum Etilefrin besteht.

5. Medikamentenkombinationen zur Herstellung eines Arzneimittels für die Behandlung der erektilen Dysfunktion gemäss Anspruch 1, wobei der Phosphodiesterase 5 Inhibitor aus Sildenafil besteht und die zweite Substanz aus dem alpha Sympathicomimetikum Oxylofrin besteht.

6. Medikamentenkombinationen zur Herstellung eines Arzneimittels für die Behandlung der erektilen Dysfunktion gemäss Anspruch 1, wobei der Phosphodiesterase 5 Inhibitor aus Sildenafil besteht und die zweite Substanz aus dem alpha Sympathicomimetikum Pholedrin besteht

7. Medikamentenkombinationen zur Herstellung eines Arzneimittels für die Behandlung der erektilen Dysfunktion gemäss Anspruch 1, wobei der Phosphodiesterase 5 Inhibitor aus Sildenafil besteht und die zweite Substanz aus dem alpha Sympathicomimetikum Ergotoxin besteht.

8. Medikamentenkombinationen zur Herstellung eines Arzneimittels für die Behandlung der erektilen Dysfunktion gemäss Anspruch 1, wobei der Phosphodiesterase 5 Inhibitor aus Sildenafil besteht und die zweite Substanz aus den Dihydroderivaten des alpha Sympathicomimetikum Ergotoxin besteht.

9. Medikamentenkombinationen zur Herstellung eines Arzneimittels für die Behandlung der erektilen Dysfunktion gemäss Anspruch 1, wobei der Phosphodiesterase 5 Inhibitor aus Sildenafil besteht und die zweite Substanz aus dem alpha Sympathicomimetikum Phenylephrin besteht.

10. Medikamentenkombinationen zur Herstellung eines Arzneimittels für die Behandlung der erektilen Dysfunktion gemäss Anspruch 1, wobei der Phosphodiesterase 5 Inhibitor aus Sildenafil besteht und die zweite Substanz aus dem alpha Sympathicomimetikum Norfenefrin besteht.

11. Medikamentenkombinationen zur Herstellung eines Arzneimittels für die Behandlung der erektilen Dysfunktion gemäss Anspruch 1, wobei der Phosphodiesterase 5 Inhibitor Sildenafil über eine Ester- oder Amidbindung mit dem jeweiligen alpha Sympathicomimetikum verbunden ist und als Syntheseprodukt in der Medikamentenkombination enthalten ist

12. Medikamentenkombinationen zur Herstellung eines Arzneimittels für die Behandlung der erektilen Dysfunktion gemäss Anspruch 1-11, **dadurch gekennzeichnet, dass** zusätzliche Komponenten aus Hüllen und oder Mantelanteilen enthalten sind, die entsprechend ihren Mischungsverhältnissen Speichel-, Magen-, Duodenal- und Dünndarmsaft-resistent sind und dadurch eine zeitlich steuerbare Freisetzung der Einzelkomponenten ermöglichen.

13. Medikamentenkombinationen zur Herstellung eines Arzneimittels für die Behandlung der erektilen Dysfunktion gemäss Anspruch 1-11, **dadurch gekennzeichnet, dass** die Wirkstoffe in Hilfsmittel integriert sind, so dass die Freisetzung zeitlich verzögert erfolgt.

14. Medikamentenkombinationen zur Herstellung eines Arzneimittels für die Behandlung der erektilen Dysfunktion nach einem der Ansprüche 1-11, **dadurch gekennzeichnet, dass** die Wirkstoffe ohne Auflösung der Kapsel zeitlich verzögert herausgelöst werden, und die Kapsel wirkstofffrei ausgeschieden wird.

15. Verwendung der Medikamentenkombination nach einem der Ansprüche 1- 14 in Hautcremes, Gel, Lotionen, Tabletten, Dragees und Injektionslösungen.

## Claims

1. A drug combination for the synthesis of pharmaceutical compounds for the treatment of erectile dysfunction (ED) in specifically diseased patients comprising a substance-family or an equivalent effector part of the synthetic compound that achieves the inhibition of phosphodiesterase subtype 5 and a second substance-family or an equivalent effector part of the synthetic compound derived from alpha Sympaticomimetics ( alpha adrenergic agonists compounds).

2. A drug combination for the synthesis of pharmaceutical compounds for the treatment of erectile dysfunction (ED) in specifically diseased patients as defined under claim1 comprising a substance-family or an equivalent effector part of the synthetic compound that achieves the inhibition of phosphodiesterase subtype 5 by Sildenafil and a second substance-family or an equivalent effector part of the synthetic compound derived from alpha Sympaticomimetics ( alpha adrenergic agonists compounds).

3. A drug combination for the synthesis of pharmaceutical compounds for the treatment of erectile dysfunction (ED) in specifically diseased patients as defined under claim1 comprising a substance-family or an equivalent effector part of the synthetic compound that achieves the inhibition of phosphodiesterase subtype 5 by Sildenafil and a second substance-family or an equivalent effector part of the synthetic compound derived from alpha Sympaticomimetics as Midodrin.

4. A drug combination for the synthesis of pharmaceutical compounds for the treatment of erectile dysfunction (ED) in specifically diseased patients as defined under claim1 comprising a substance-family or an equivalent effector part of the synthetic compound that achieves the inhibition of phosphodiesterase subtype 5 by Sildenafil and a second substance-family or an equivalent effector part of the synthetic compound derived from alpha Sympaticomimetics as Etilefrin.

5. A drug combination for the synthesis of pharmaceutical compounds for the treatment of erectile dysfunction (ED) in specifically diseased patients as defined under claim1 comprising a substance-family or an equivalent effector part of the synthetic compound that achieves the inhibition of phosphodiesterase subtype 5 by Sildenafil and a second substance-family or an equivalent effector part of the synthetic compound derived from alpha Sympaticomimetics as Oxylofrin.

6. A drug combination for the synthesis of pharmaceutical compounds for the treatment of erectile dysfunction (ED) in specifically diseased patients as defined under claim 1 comprising a substance-family or an equivalent effector part of the synthetic compound that achieves the inhibition of phosphodiesterase subtype 5 by Sildenafil and a second substance-family or an equivalent effector part of the synthetic compound derived from alpha Sympaticomimetics as Pholedrin

7. A drug combination for the synthesis of pharmaceutical compounds for the treatment of erectile dysfunction (ED) in specifically diseased patients as defined under claim 1 comprising a substance-family or an equivalent effector part of the synthetic compound that achieves the inhibition of phosphodiesterase subtype 5 by Sildenafil and a second substance-family or an equivalent effector part of the synthetic compound derived from alpha Sympaticomimetics as Ergotoxin.

8. A drug combination for the synthesis of pharmaceutical compounds for the treatment of erectile dysfunction (ED) in specifically diseased patients as defined under claiml comprising a substance-family or an equivalent effector part of the synthetic compound that achieves the inhibition of phosphodiesterase subtype 5 by Sildenafil and a second substance-family or an equivalent effector part of the synthetic compound derived from alpha Sympaticomimetics as dihydroderivatives from Ergotoxin.

9. A drug combination for the synthesis of pharmaceutical compounds for the treatment of erectile dysfunction (ED) in specifically diseased patients as defined under claiml comprising a substance-family or an equivalent effector part of the synthetic compound that achieves the inhibition of phosphodiesterase subtype 5 by Sildenafil and a second substance-family or an equivalent effector part of the synthetic compound derived from alpha Sympaticomimetics as Phenylephrin

10. A drug combination for the synthesis of pharmaceutical compounds for the treatment of erectile dysfunction (ED) in specifically diseased patients as defined under claiml comprising a substance-family or an equivalent effector part of the synthetic compound that achieves the inhibition of phosphodiesterase subtype 5 by Sildenafil and a second substance-family or an equivalent effector part of the synthetic compound derived from alpha Sympaticomimetics as Norfenefrin.

11. A drug combination for the synthesis of pharmaceutical compounds for the treatment of erectile dysfunction (ED) in specifically diseased patients as defined under claim1 comprising a substance-family or an equivalent effector part of the synthetic compound that achieves the inhibition of phosphodiesterase subtype 5 by Sildenafil and a second substance-family or an equivalent effector part of the synthetic compound derived from alpha Sympaticomimetics synthesized by Ester-or Amid linkages.

12. A drug combination for the synthesis of pharmaceutical compounds for the treatment of erectile dysfunction (ED) in specifically diseased patients as defined under claims1-11 further **characterized, that** additional coatings components are fabricated into drug formulations covering all types of standards resistant against saliva, gastric, duodenal intestinal juice digestion. This results in a controlled release of the drug components and guarantees a time-correlated release of the effective drugs from the synthetic drug composed of two different pharmacologically active compounds.

13. A drug combination for the synthesis of pharmaceutical compounds for the treatment of erectile dysfunction (ED) in specifically diseased patients as defined under claims1-11 further **characterized, that** additional components are fabricated into drug formulations resulting in a extended release of the drug components.

14. A drug combination for the synthesis of pharmaceutical compounds for the treatment of erectile dysfunction (ED) in specifically diseased patients as defined under claims1-11 further **characterized, that** active components of drug formulations can be timed extended released without destruction of the tablets or capsules. This results in a controlled release of the drug components and guarantees a drug-free discharge of the tablets or capsules.

15. Application of the drug combination as defined under claims 1-14 in creams, lotios, tablets, capsules, and injectables

## Revendications

1. Des combinaisons de substances pour produire un médicament contre la dysfonction érectile contenant une substance qui inhibe la Phosphodiesterase type 5 et une seconde qui est un alpha-Sympathicomimétique.

2. Des combinaisons de substances pour produire un médicament contre la dysfonction érectile selon prétention 1, étant l'inhibiteur de la Phosphodiesterase type 5 le Sildenafil et la seconde substance un alpha-Sympathicomimétique.

3. Des combinaisons de substances pour produire un médicament contre la dysfonction érectile selon prétention 1, étant l'inhibiteur de la Phosphodiesterase type 5 le Sildenafil et la seconde substance l'alpha-Sympathicomimétique Midodrine.

4. Des combinaisons de substances pour produire un médicament contre la dysfonction érectile selon prétention 1, étant l'inhibiteur de la Phosphodiesterase type 5 le Sildenafil et la seconde substance l'alpha-Sympathicomimétique Etilefrine

5. Des combinaisons de substances pour produire un médicament contre la dysfonction érectile selon prétention 1, étant l'inhibiteur de la Phosphodiesterase type 5 le Sildenafil et la seconde substance l'alpha-Sympathicomimétique Oxilofrine.

6. Des combinaisons de substances pour produire un médicament contre la dysfonction érectile selon prétention 1, étant l'inhibiteur de la Phosphodiesterase type S le Sildenafil et la seconde substance l'alpha-Sympathicomimétique Pholedrine.

7. Des combinaisons de substances pour produire un médicament contre la dysfonction érectile selon prétention 1, étant l'inhibiteur de la Phosphodiesterase type 5 le Sildenafil et la seconde substance l'alpha-Sympathicomimétique Ergotoxine.

8. Des combinaisons de substances pour produire un medicament contre la dysfonction érectile selon prétention 1, étant l'inhibiteur de la Phosphodiesterase type 5 le Sildenafil et la seconde substance des dihydrodérivés de l'alpha-Sympathicomimétique Ergotoxine.

9. Des combinaisons de substances pour produire un médicament contre la dysfonction érectile selon prétention 1, étant l'inhibiteur de la Phosphodiesterase type 5 le Sildenafil et la seconde substance l'alpha-Sympathicomimétique Phenylephrine.

10. Des combinaisons de substances pour produire un médicament contre la dysfonction érectile selon prétention 1, étant l'inhibiteur de la Phosphodiesterase type 5 le Sildenafil et la seconde substance l'alpha-Sympathicomimétique Norfenofrine.

11. Des combinaisons de substances pour produire un médicament contre la dysfonction érectile selon prétention 1, étant l'inhibiteur de la Phosphodiesterase type 5 le Sildenafil lié par des liaisons amides ou esters au alpha-Sympathicomimétique respectif et est compris comme produit de synthèse dans la combinaison.

12. Des combinaisons de substances pour produire un medicament contre la dysfonction érectile selon prétentions 1 - 11, étant **caractérisés par le fait qu'**il y a en plus des parts d'enveloppes ou de revêtements qui selon leur proportions de mélange sont résistants à la salive ou au suc gastrique ou au suc duodénal ou au suc intestinal et permettent par cela de dégager les composants l'un après l'autre en ordre chronologique.

13. Des combinaisons de substances pour produire un médicament contre la dysfonction érectile selon prétentions 1 - 11, **caractérisés par le fait que** les principes actifs sont intégrés dans des matérielles auxiliaires alors que le dégagement se fait avec du retardement.

14. Des combinaisons de substances pour produire un médicament contre la dysfonction érectile selon prétentions 1 - 11, **caractérisés par le fait que** les principes actifs sont dégagés avec du retardement de la capsule sans que celle - ci est dissolue et que la capsule est éliminée sans les principes actifs.

15. Utilisation des combinaisons selon prétentions 1 - 14 dans des crèmes pour la peaux, des gels, des lotions, des comprimés, des dragées et des liquides injectables.
